# EUROPÄISCHE PATENTANMELDUNG

(11) **EP 1 284 129 A1**
(43) Veröffentlichungstag der Anmeldung: **19.02.2003**
(21) Anmeldenummer: 02016605.4
(22) Anmeldetag: 25.07.2002
(51) Int. Cl.: A61K 7/42, A61K 7/48

(54) **Kosmetische und dermatologische Lichtschutzformulierungen mit einem Gehalt an wasserlöslichen UV-Filtersubstanzen und Iminodibernsteinsäure und/oder ihren Salzen**

(30) Priorität: 17.08.2001 DE 10140540
(71) Anmelder: Beiersdorf AG, 20245 Hamburg (DE)
(72) Erfinder: Göppel, Anja, 22527 Hamburg (DE); Kranz, Ariane, 20251 Hamburg (DE); Dörschner, Albrecht, 20146 Hamburg (DE); Kröpke, Rainer, 22869 Schenefeld (DE)

(57) **Zusammenfassung**

Lichtschutzwirksame kosmetische oder dermatologische Zubereitungen, dadurch gekennzeichnet, daß sie
(a) mindestens eine wasserlösliche UV-Filtersubstanz und
(b) Iminodibernsteinsäure und/oder ihre Salze
enthalten und deren Verwendung.

## Beschreibung

Die vorliegende Erfindung betrifft kosmetische und dermatologische Lichtschutzzubereitungen, insbesondere betrifft sie sandabweisende kosmetische und dermatologische Lichtschutzzubereitungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich beispielsweise um Derivate des 3-Benzylidencamphers, der 4-Aminobenzoesäure, der Zimtsäure, der Salicylsäure, des Benzophenons sowie des s-Triazins handelt.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.
So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, daß kosmetische und dermatologische Lichtschutzzubereitungen sowohl gegen UV-B- als auch gegen UV-A-Strahlung ausreichenden Schutz bieten.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen.

Die Einsatzkonzentration bekannter als Feststoff vorliegender Lichtschutzfiltersubstanzen ist allerdings häufig - gerade in Kombination mit anderen zu lösenden Substanzen - begrenzt. Es bereitet daher gewisse formulierungstechnische Schwierigkeiten, höhere Lichtschutzfaktoren bzw. UV-A-Schutzleistung zu erzielen.

Der Sonnenbrand bzw. das Lichterythem sind die akuten Erscheinungsformen der Lichteinwirkung. Neben den bereits beschriebenen Wirkungen der UV-Strahlen kommt es in der Nachreaktion der Haut ferner zu einer verminderten Sebumproduktion und einem Austrocknen der Haut. Zur Linderung und zur Pflege dieser Phänomene kennt der Stand der Technik Produkte, welche nach dem Sonnenbaden angewendet werden und üblicherweise spezielle Wirkstoffe enthalten, wie beispielsweise
■ Rückfettungs- und Feuchthaltemittel,
■ entzündungslindernde und kühlende Stoffe,
■ lokal anaestesierende Stoffe und/oder
■ desinfizierende Stoffe, um mögliche Hautinfektionen zu verhindern.

Diese sogenannten Aftersun- oder Après-soleil-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Allerdings mangelt es dem Stand der Technik an Produkten, welche die Haut bereits während der UV-Einstrahlung vor dem Austrocknen schützen und sie ausreichend pflegen.

Ein weiterer Nachteil des Standes der Technik ist, daß übliche Lichtschutzformulierungen einen meist klebrigen Film auf der Haut hinterlassen. Dies hat z. B. bei der Anwendung solcher Produkte an einem Sandstrand zur Folge, daß der Sand am Körper haften bleibt, was vom Anwender als unangenehm empfunden wird und im schlimmsten Fall dazu führen kann, daß das Sonnenschutzmittel zu wenig oder gar nicht mehr verwendet wird. Da am Meer meist ein mehr oder weniger starker Wind herrscht, tritt dieser Nachteil in der Regel selbst dann auf, wenn der Körper gar nicht direkt mit dem Sand in Berührung kommt - beispielsweise beim Sonnenbaden auf einem Liegestuhl - da auch der im Wind herumwirbelnde Sandstaub auf den eingecremten Hautpartien haften bleibt.

Eine weitere Aufgabe der vorliegenden Erfindung war daher, Lichtschutzformulierungen zu finden, nach deren Anwendung kein Sand auf der eingecremten Haut kleben bleibt, die also dementsprechend als sandabweisend zu bezeichnen sind.

Es war überraschend und für den Fachmann nicht vorauszusehen, daß
lichtschutzwirksame kosmetische oder dermatologische Zubereitungen, dadurch gekennzeichnet, daß sie
(a) mindestens eine wasserlösliche UV-Filtersubstanz und
(b) Iminodibernsteinsäure und/oder ihre Salze
   enthalten,
den Nachteilen des Standes der Technik abhelfen.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion, eine Feststoff-Emulsionen (d. h. eine Emulsion, welche durch Feststoffe stabilisiert ist, z. B. eine Pickering-Emulsion), eine sprühbare Emulsion oder eine Hydrodispersion sein.

Die erfindungsgemäßen Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische und kosmetische Eigenschaften, wie beispielsweise die Verteilbarkeit auf der Haut oder das Einzugsvermögen in die Haut, und zeichen sich ferner durch eine sehr gute Lichtschutzeffektivität bei gleichzeitig hervorragenden Hautpflegedaten aus.

Gegenstand der Erfindung sind daher auch
lichtschutzwirksame kosmetische oder dermatologische Zubereitungen, dadurch gekennzeichnet, daß sie synergistische Stoffkombinationen von
(a) mindestens einer wasserlöslichen UV-Filtersubstanz und
(b) Iminodibernsteinsäure und/oder ihren Salzen
   enthalten,
   wobei die UV-Schutzleistung dieser Zubereitungen höher ist als die gleicher Zubereitungen, welche keine Substanzen gemäß (b) enthalten.

Die UV-Schutzleistung von Sonnenschutzmittel bzw. der ihnen zugrunde liegenden UV-Filter wird in der Regel in biologischen Wirksamkeitsprüfungen unter standardisierten Bedingungen bestimmt. Mit "UV-Schutzleistung" ist im Sinne der vorliegenden Erfindung sowohl die Schutzleistung gegenüber UV-A-Strahlung als auch gegenüber UV-B-Strahlung gemeint.

Ein Maß für die UV-Schutzleistung stellen im Sinne der vorliegenden Erfindung beispielsweise der Lichtschutzfaktor (LSF bzw. SPF) oder auch IPD-Werte und dergleichen dar.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich von 320 bis 360 nm absorbieren.

Die kosmetischen und dermatologischen Zubereitungen im Sinne der vorliegenden Erfindung hinterlassen auf der Haut keinen schmierigen oder klebrigen Eindruck, sind ausgezeichnet hautverträglich und zeichnen sich ferner überaschender Weise dadurch aus, daß sie sandabweisend sind.

Gegenstand der Erfindung sind daher auch
sandabweise kosmetische oder dermatologische Lichtschutzzubereitungen, dadurch gekennzeichnet, daß sie
(a) mindestens eine wasserlösliche UV-Filtersubstanz und
(b) Iminodibernsteinsäure und/oder ihre Salze
enthalten.

Vorteilhafte wasserlösliche UV-Filtersubstanzen im Sinne der vorliegenden Erfindung sind sulfonierte UV-Filter, insbesondere:
Phenylen-1,4-bis-(2-benzimidazyl)-3,3'-5,5'-tetrasulfonsäure, welche sich durch folgende Struktur auszeichnet: 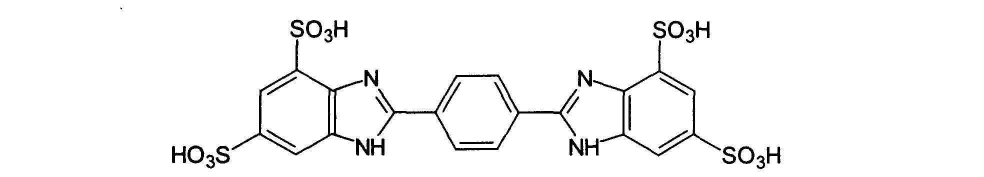 sowie ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 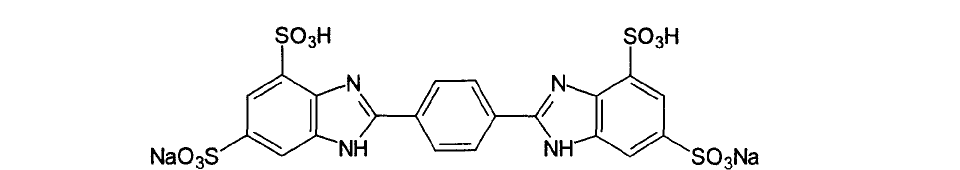 mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist.

Weitere vorteilhafte sulfonierte UV-Filter im Sinne der vorliegenden Erfindung sind die Salze der 2-Phenylbenzimidazol-5-sulfonsäure, wie ihr Natrium-, Kalium- oder ihr Triethanolammonium-Salz sowie die Sulfonsäure selbst 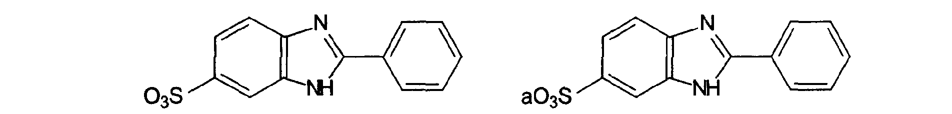 mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist.

Ferner vorteilhaft im Sinne der vorliegenden Erfindung sind das 1,4-di(2-oxo-10-Sulfo-3-bornylidenmethyl)-Benzol (auch: 3,3'-(1,4-Phenylendimethylene)-bis-(7,7-dimethyl-2-oxobicyclo-[2.2.1]hept-1-ylmethan Sulfonsäure) und dessen Salze (besonders die entprechenden 10-Sulfato-verbindungen, insbesondere das entsprechende Natrium-, Kaliumoder Triethanolammonium-Salz), das auch als Benzol-1,4-di(2-oxo-3-bornylidenmethyl-10-sulfonsäure) bezeichnet wird und sich durch die folgende Struktur auszeichnet:

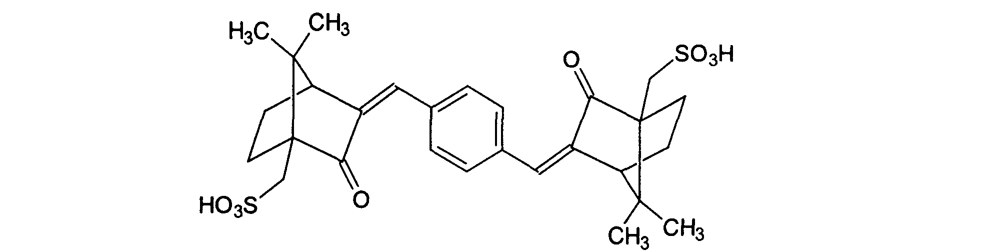

Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich.

Weitere vorteilhafte wasserlösliche UV-B- und/oder Breitband-Filtersubstanzen im Sinne der vorliegenden Erfindung sind z. B. Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Die Gesamtmenge an einer oder mehreren wasserlöslichen UV-Filtersubstanzen in den fertigen kosmetischen oder dermatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen.

Iminodibernsteinsäure hat folgende Struktur, wobei vermutlich ein Gleichgewicht tautomerer Formen vorliegt:

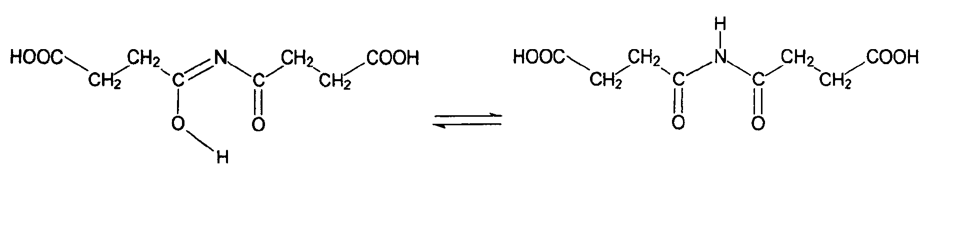

Iminodibersteinsäure ist u.a. von der Fa. Bayer AG unter den Handelsnamen Iminodisuccinat VP OC 370 (ca. 30% ige Lösung) und Baypure CX 100 fest erhältlich.

Erfindungsgemäß enthalten kosmetische oder dermatologische Zubereitungen 0,001 bis 15 Gew.-%, vorteilhaft 0,01 bis 10 Gew.-%, ganz besonders bevorzugt 0,05 bis 5 Gew.-% Iminodibernsteinsäure und/oder ihrer Salze.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder ß-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Femer ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 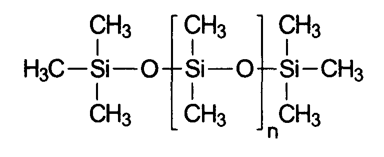 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z.B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |
| Tioveil AQ 10PG | Alumina / Silica | Solaveil / Uniquema |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- Dioctylbutylamidotriazon (INCI: Dioctylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich sein. Vorteilhafte öllösliche Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure®-Typen von Goodrich), Dimethicone Copolyol Polyacrylate (Silsoft Surface® von der Witco Organo Silicones Group), PVPNA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

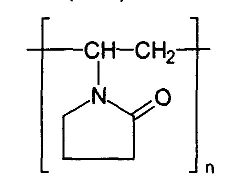

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### 1. O/W Sonnenschutz Emulsionen

| | **1** | **2** | **3** | **4** | **5** | **6** | **7** |
|---|---|---|---|---|---|---|---|
| Glycerinmonostearat SE | | | 0,50 | 1,00 | 3,00 | 1,50 | |
| Glyceryl Stearat Citrat | 2,00 | | 3,50 | | 2,00 | | 2,50 |
| Stearinsäure | | 3,00 | | 2,00 | | | |
| PEG-40 Stearate | 0,50 | | | | | 2,00 | |
| PEG-100 Stearate | | 1,50 | | 0,25 | | | |
| Trilaureth-4 Phosphate | | 0,50 | | | 1,00 | | |
| Cetearyl Sulfat | | | 2,00 | | | 2,00 | 0,50 |
| Cetyl Alkohol | | 1,00 | | 1,50 | | | 2,00 |
| Aniso Triazin | | 1,50 | | 2,00 | 2,50 | | 2,50 |
| Butyl Methoxydibenzoylmethan | 2,00 | | 2,00 | | | | |
| Mexoryl XL ® | | | | | 2,50 | | |
| Diethylhexyl Butamidotriazon | | 2,00 | | 2,00 | | | |
| Ethylhexyl Triazon | 4,00 | | 3,00 | | | 2,00 | |
| Ethylhexyl Methoxycinnamat | | | | 5,00 | 6,00 | | 8,00 |
| Octocrylen | | 4,00 | | | | | 2,50 |
| Mexoryl SX® | | 1,00 | | | 0,25 | | |
| Bisimidazylat | 1,00 | | 0,50 | | | 1,00 | 1,50 |
| Phenylbenzmidazol Sulfon-säure | 0,50 | | | 3,00 | | | |
| Titandioxid MT-100 TV ® | 1,00 | 1,50 | | 3,00 | | 2,00 | |
| Zinkoxid Z-Cote ® | | | | | 5,00 | | |
| C12-15 Alkyl Benzoate | | 2,50 | | | 4,00 | 7,00 | |
| Dicaprylyl Ether | | | 3,50 | | 2,00 | | |
| Dicaprylyl Carbonat | 5,00 | | | 9,00 | | 2,00 | |
| Dimethicon | | 0,50 | | | 2,00 | | 2,0 |
| Cyclomethicon | 2,00 | | | 0,50 | | | |
| PVP Hexadecen Copolymer | 0,50 | | | 0,50 | | | 1,00 |
| Silsoft Surface ® | | 1,00 | 2,50 | | | | 0,75 |
| Glycerin | 3,00 | 7,50 | | 7,50 | 5,00 | | 2,50 |
| Xanthan Gummi | 0,15 | | 0,05 | | | | 0,30 |
| Vitamin E Acetat | 0,50 | | 0,25 | | 0,75 | | 1,00 |
| Baypure CX 100 ® | 0,30 | 0,50 | | | 0,15 | | 0,60 |
| Imiosuccinat VP OC 370 ® | | | 1,00 | 1,20 | | 0,75 | |
| EDTA | 0,10 | | | | | 0,30 | |
| NaOH-Lsg. (50%) | | 0,13 | | 0,20 | | | |
| Glydant ® | | 0,60 | 0,40 | | | | |
| Glycacil - S® | | | | 0,18 | 0,20 | 0,10 | 0,15 |
| Methylparaben | 0,15 | | 0,25 | | 0,50 | | |
| Phenoxyethanol | 1,00 | 0,40 | | 0,40 | 0,50 | 0,40 | 0,60 |
| Ethanol | | 2,00 | 1,50 | | 3,00 | | 1,00 |
| Parfüm | 0,20 | | | | 0,20 | 0,20 | 0,20 |
| Wasser | ad 100 | ad 100 | ad 100 | ad 100 | ad 100 | ad 100 | ad 100 |

### 2. Hydrodispersionen

| | **1** | **2** | **3** | **4** | **5** |
|---|---|---|---|---|---|
| Ceteareth-20 | 1,00 | | | 0,5 | |
| Cetyl Alkohol | | | 1,00 | | |
| Sodium Carbomer | | 0,20 | | 0,30 | |
| Acrylates/C10-30 Alkyl Acrylate Crosspolymer | 0,50 | | 0,40 | 0,10 | 0,10 |
| Cetearyl Sulfat | | 0,50 | | | |
| Xanthan Gummi | | 0,30 | 0,15 | | 0,50 |
| Ethylhexyl Methoxycinnamat | | | | 5,00 | 8,00 |
| Aniso Triazin | | 1,50 | | 1,00 | 2,50 |
| Butyl Methoxydibenzoylmethan | 1,00 | | 2,00 | 0,50 | |
| Diethylhexyl Butamidotriazon | | 2,00 | | 2,00 | 1,00 |
| Ethylhexyl Triazon | 4,00 | | 3,00 | 4,00 | |
| 4-Methylbenzylidene Camphor | 4,00 | 4,00 | | | 2,00 |
| Octocrylen | | 4,00 | 4,00 | | 2,50 |
| Bisimidazylate | 1,00 | | 0,50 | | 2,00 |
| Mexoryl SX ® | | 0,25 | | 1,00 | |
| Phenylbenzmidazole Sulfonsäure | 0,50 | | | 3,00 | |
| Tioveil AQ 10PG® | 0,50 | | 2,00 | 3,00 | 1,00 |
| C12-15 Alkyl Benzoate | 2,00 | 2,50 | | | |
| Butylenglycol Dicaprylat/Dicaprat | 4,00 | | 2,00 | 6,00 | |
| Dicaprylyl Carbonat | | 2,00 | 6,00 | | |
| Dimethicon | | 0,50 | 1,00 | | |
| Distarch Phosphat | 2,00 | | 1,50 | | |
| Vaseline | | 1,50 | | | |
| PVP Hexadecen Copolymer | 0,50 | | | 0,50 | 1,00 |
| Polyurethan-4 | | 0,25 | | | 1,00 |
| Styrene/Acrylat Copolymer | 1,00 | | 0,50 | | |
| Ethylhexyloxyglycerin | | 0,50 | 1,00 | | 0,50 |
| Glycerin | 3,00 | 7,50 | | 7,50 | 2,50 |
| Vitamin E Acetat | 0,50 | 0,20 | 0,25 | 0,75 | 1,00 |
| α - Glycosil Rutin | | 0,30 | | 0,25 | |
| Baypure CX 100 ® | 0,40 | 0,50 | | | 0,20 |
| Imiosuccinat VP OC 370 ® | | | 1,50 | 0,75 | |
| EDTA | | | | 0,20 | |
| Glydant 2000® | | | 0,40 | 0,20 | 0,10 |
| Konkaben LMB® | 0,20 | | | | 0,15 |
| Methylparaben | 0,50 | | 0,25 | 0,15 | |
| Phenoxyethanol | 0,50 | | | 1,00 | 0,60 |
| Ethanol | 3,00 | 7,00 | | | 1,00 |
| Wasserlöslicher Farbstoff | q.s. | q.s. | q.s. | q.s. | q.s. |
| Parfüm | 0,20 | | 0,20 | | 0,20 |
| Wasser | ad. 100 | ad. 100 | ad. 100 | ad. 100 | ad. 100 |

### 3. W/O Sonnenschutz Emulsionen

| | **1** | **2** | **3** | **4** | **5** |
|---|---|---|---|---|---|
| Cetyldimethicon Copolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| Ethylhexyl Methoxycinnamat | | 8,00 | | 5,00 | 4,00 |
| Aniso Triazin | 2,00 | | | 2,00 | 2,50 |
| Butyl Methoxydibenzoylmethan | | | 2,00 | 1,00 | |
| Diethylhexyl Butamidotriazon | 3,00 | 1,00 | | | 3,00 |
| Ethylhexyl Triazon | | | 3,00 | 4,00 | |
| Mexoryl XL | | 2,00 | | 4,00 | 2,00 |
| Octocrylen | 7,00 | 2,50 | 4,00 | | 2,50 |
| Bisimidazylat | 1,00 | 2,00 | 0,50 | | |
| Mexoryl SX ® | 0,75 | | 0,20 | | |
| Phenylbenzmidazol Sulfonsäure | 0,50 | | | 3,00 | 2,00 |
| MT-100 Z ® | | 2,00 | 1,50 | | 3,00 |
| Zinkoxid Z-Cote HP1® | 2,50 | | 6,00 | | |
| Mineralöl | | 10,0 | | | 8,50 |
| C12-15 Alkyl Benzoate | | | | 9,00 | |
| Coco Glycerides | 10,00 | | | | 7,00 |
| Dicaprylyl Carbonat | | 7,00 | 2,00 | 8,00 | 4,00 |
| Dimethicon | | | | 5,00 | |
| Cyclomethicon | 6,00 | | | | 2,00 |
| PVP Hexadecene Copolymer | 0,50 | | | 0,50 | 1,00 |
| Baypure CX 100® | 0,45 | | 0,75 | | 0,25 |
| Imiosuccinat VP OC 370 ® | | 2,00 | | 1,00 | |
| Ethylhexyloxyglycerin | | 0,30 | 1,00 | | 0,50 |
| Glycerin | 3,00 | 7,50 | | 7,50 | 2,50 |
| Glycine Soja | | 1,00 | 1,50 | | |
| MgSO₄ | 1,00 | 0,50 | | 0,50 | |
| Vitamin E Acetat | 0,50 | | 0,25 | | 1,00 |
| DMDM Hydantoin | | 0,60 | 0,40 | 0,20 | |
| Methylparaben | 0,50 | | 0,25 | 0,15 | |
| Phenoxyethanol | 0,50 | 0,40 | | 1,00 | |
| Ethanol | 3,00 | | 1,50 | | 1,00 |
| Parfüm | 0,20 | | | 0,20 | 0,20 |
| Wasser | ad. 100 | ad. 100 | ad. 100 | ad. 100 | ad. 100 |

### 4. PIT - Sonnen Sprays

| | **1** | **2** | **3** | **4** | **5** |
|---|---|---|---|---|---|
| Glycerinmonostearat SE | 0,50 | | 3,00 | 2,00 | 4,00 |
| Ceteareth-12 | | 5,00 | | 1,00 | 1,50 |
| Ceteareth-20 | | | | 2,00 | |
| Steareth-20 | 5,00 | | 1,00 | | |
| Cetyl Palmitat | | | 2,00 | | 0,50 |
| Cetyl Phosphat | | 0,25 | | | |
| Cetyl Alkohol | 2,50 | 1,00 | | 1,50 | |
| Cetyldimethicon Copolyol | | | | 0,75 | 0,50 |
| Mexoryl XL® | 0,25 | | 1,00 | | |
| Ethylhexyl Methoxycinnamat | 2,00 | | | 5,00 | 8,00 |
| Aniso Triazin | | 1,50 | | 2,00 | 2,50 |
| Butyl Methoxydibenzoylmethan | | 0,50 | 2,00 | | |
| Diethylhexyl Butamidotriazon | 1,00 | 2,00 | | 0,50 | |
| Ethylhexyl Triazon | | | 3,00 | 4,00 | |
| Octocrylen | 5,00 | 4,00 | | | 2,50 |
| Bisimidazylat | 3,00 | 1,00 | 0,50 | | 1,50 |
| Mexoryl SX ® | | 0,75 | | 1,50 | |
| Phenylbenzmidazol Sulfonsäure | 0,50 | | 2,00 | 3,00 | |
| C12-15 Alkyl Benzoat | | 2,50 | | | 5,00 |
| Coco Glyceride | 2,50 | | | 4,00 | |
| Dicaprylyl Ether | | | 3,50 | | |
| Butylenglycol Dicaprylat/Dicaprat | 5,00 | | | 6,00 | |
| Dicaprylyl Carbonat | | | 6,00 | | 2,00 |
| Silsoft Surface ® | 2,00 | | | 0,50 | 0,50 |
| Polyurethan-4 | | 0,75 | | 0,50 | |
| PVP Hexadecen Copolymer | 0,50 | | | 0,50 | 1,00 |
| Glycerin | | 7,50 | 5,00 | 7,50 | 2,50 |
| Reines Vitamin E | 0,50 | | 0,25 | | 1,00 |
| Baypure CX 100® | 0,40 | 0,50 | | | 0,20 |
| Imiosuccinat VP OC 370 ® | | | 1,50 | 0,75 | |
| EDTA | | | | 0,20 | |
| DMDM Hydantoin | 0,60 | | 0,40 | 0,20 | |
| Glycacil - L ® | | 0,20 | | | 0,15 |
| Methylparaben | | 0,50 | 0,25 | 0,15 | |
| Phenoxyethanol | 0,50 | 0,40 | | 1,00 | 0,60 |
| Ethanol | | 2,00 | 1,50 | | 1,00 |
| Parfüm | 0,20 | | 0,20 | | 0,20 |
| Wasser | ad. 100 | ad. 100 | ad. 100 | ad. 100 | ad. 100 |

### 5. Feststoffstablisisierte Emulsionen

| | **1** | **2** | **3** | **4** | **5** |
|---|---|---|---|---|---|
| Mineralöl | | 4,0 | 16,0 | 16,0 | |
| Octyldodecanol | 9,0 | | 5,0 | | |
| Caprylic/Capric Triglycerid | 9,0 | 9,0 | 6,0 | | |
| C12-15- Alkyl Benzoate | | | | 5,0 | 8,0 |
| Butylene Glycol Caprylate/Caprate | | | | | 8,0 |
| Dicaprylyl Ether | 9,0 | | | 4,0 | |
| Dicaprylyl Carbonate | | 9,0 | | | |
| Hydroxyoctacosanyl Hydroxystearate | | 2,0 | 2,0 | 2,0 | 1,5 |
| Disteardimonium Hectorit | 3,0 | 0,75 | 0,5 | | 0,25 |
| Cera Microcristallina + Paraffinum Liquidum | | 2,0 | | | 5,0 |
| Hydroxypropyl Methylcellulose | | | | | 0,05 |
| Cyclomethicon | | 2,5 | | | 3,0 |
| Ethylhexylmethoxycinnamat | | | 7,5 | 6,0 | |
| Diethylhexyl Butamido Triazon | | 2,0 | | | 4,0 |
| Tinosorb M ® | 0,5 | | | | 4,0 |
| Bisimidazylat | 1,0 | 2,5 | 0,5 | | |
| Mexoryl SX ® | | 1,0 | | 1,5 | |
| Phenylbenzmidazol Sulfonsäure | | | 3,0 | 0,5 | 2,0 |
| Octocrylen | 2,5 | 5,0 | | | |
| Eusolex T-2000 ® | | | 4,0 | 2,0 | 4,0 |
| Titandioxid T 805 ® | 1,0 | | | | 3,0 |
| Z-Cote HP1 ® | 3,0 | 2,0 | | 6,0 | |
| Silica Dimethyl Silylat | | | 1,0 | | |
| Bornitrid | 2,0 | 1,0 | | 1,50 | |
| Stärke/-Natriummetaphosphat-Polymer | | 2,0 | | | |
| Tapioca Stärke | | | | 1,0 | |
| Natrium Chlorid | 1,0 | 1,0 | 1,0 | 1,0 | 1,0 |
| Glycerin | 5,0 | 10,0 | 3,0 | 6,0 | 10,0 |
| Baypure CX 100® | 0,40 | 0,50 | | | 0,20 |
| Imiosuccinat VP OC 370 ® | | | 1,50 | 0,75 | |
| Trinatrium EDTA | | 1,0 | | 1,0 | |
| Methylparaben | 0,21 | | | | 0,2 |
| Propylparaben | 0,07 | | | | |
| Phenoxyethanol | 0,5 | | 0,4 | 0,4 | 0,5 |
| Hexamidin Diisethionat | | | | | 0,08 |
| Diazolidinyl Harnstoff | | | 0,28 | 0,28 | |
| Alcohol | | 5,0 | | 2,5 | |
| Parfüm | q.s. | q.s. | q.s. | q.s. | q.s. |
| Wasser | ad 100 | ad 100 | ad 100 | ad 100 | ad 100 |

## Patentansprüche

1. Lichtschutzwirksame kosmetische oder dermatologische Zubereitungen, **dadurch gekennzeichnet, daß** sie
(a) mindestens eine wasserlösliche UV-Filtersubstanz und
(b) Iminodibernsteinsäure und/oder ihre Salze
enthalten.

2. Zubereitung nach Anspruch 1, **dadurch gekennzeichnet, daß** sie sandabweisend ist.

3. Lichtschutzwirksame kosmetische oder dermatologische Zubereitungen, **dadurch gekennzeichnet, daß** sie synergistische Stoffkombinationen von
(a) mindestens einer wasserlöslichen UV-Filtersubstanz und
(b) Iminodibernsteinsäure und/oder ihren Salzen
enthalten,
wobei die UV-Schutzleistung dieser Zubereitungen höher ist als die gleicher Zubereitungen, welche keine Substanzen gemäß (b) enthalten.

4. Zubereitung nach einem der Ansprüche 1 bis 3, **dadurch gekennzeichnet, daß** der Gehalt an Iminodibernsteinsäure und/oder ihren Salzen aus dem Bereich von 0,001 bis 15 Gew.-%, vorteilhaft 0,01 bis 10 Gew.-%, besonders bevorzugt 0,05 bis 5 Gew.-% gewählt wird, bezogen auf das Gesamtgewicht der Zubereitung.

5. Zubereitung nach einem der vorhergehenden Ansprüche, **dadurch gekennzeichnet, daß** sie mindestens eine weitere UV-Filtersubstanz, gewählt aus der Gruppe Triazine, Benzotriazole und organische und/oder anorganische Pigmente, enthält.

6. Zubereitung nach einem der vorhergehenden Ansprüche, **dadurch gekennzeichnet, daß** sie mindestens eine weitere UV-A-Filtersubstanz und/oder einen Breitbandfilter, gewählt aus der Gruppe Dibenzoylmethanderivate [insbesondere das 4-(tert.-Butyl)-4'-methoxydibenzoylmethan] und 2,4-Bis-{[4-(2-Ethyl-hexyloxy)-2-hydroxy]-phenyl}-6-(4-methoxyphenyl)-1,3,5-triazin, enthält, wobei die weiteren Filtersubstanzen jeweils einzeln oder in beliebigen Kombinationen miteinander vorliegen können.

7. Zubereitung nach einem der vorhergehenden Ansprüche, **dadurch gekennzeichnet, daß** sie mindestens ein Flavonglycosid, insbesondere α-Glucosylrutin, und/oder Vitamin E und/oder dessen Derivate enthält.

8. Verwendung von Zubereitungen nach einem der vorhergehenden Ansprüche zur Hautbefeuchtung.

9. Verwendung von Zubereitungen nach einem der Ansprüche 1 bis 7 zum Schutz vor lichtbedingter Hautalterung.

10. Verwendung von Stoffkombinationen von
(a) mindestens einer wasserlöslichen UV-Filtersubstanz und
(b) Iminodibernsteinsäure und/oder ihren Salzen
zur Herstellung von Zubereitungen zur Prophylaxe und Behandlung kosmetischer oder dermatologischer Hautveränderungen, wie sie bei der Hautalterung auftreten.
